# EUROPEAN PATENT APPLICATION

(11) **EP 4 792 743 A1**
(43) Date of publication of application: **19.08.2026**
(21) Application number: 25158084.1
(22) Date of filing: 14.02.2025
(51) Int. Cl.: A61B 6/00, G06T 7/11

(54) **CONSTRUCTION OF PNEUMOTHORAX IMAGES**

(71) Applicant: Koninklijke Philips N.V., 5656 AE Eindhoven (NL)
(72) Inventor: KOEHLER, Thomas, Eindhoven (NL)
(74) Representative: Philips Intellectual Property & Standards

(57) **Abstract**

Disclosed herein is a method of x-ray imaging. The method comprises receiving (200) a dark-field x-ray image (126) descriptive of a field of view (320) comprising a lung region of a subject (304).

The method further comprises receiving (202) a transmission x-ray image (124) descriptive of the field of view of the lung region of the subject. The method further comprises receiving (204) a pulmonary segmentation (128, 600, 700, 700') in response to inputting one of the dark-field x-ray image and the transmission x-ray image into a pulmonary segmentation module (122). The method further comprises constructing (206) pneumothorax image (130, 130', 130") by overlaying the pulmonary segmentation onto the other of the dark-field x-ray image and the transmission x-ray image. The method further comprises providing (208) the pneumothorax image.

## Description

### TECHNICAL FIELD

The invention relates to x-ray imaging, in particular to darkfield x-ray imaging.

### BACKGROUND

Pneumothorax is a condition where air accumulates in the pleural space between the lung and the chest wall. The pleural space and the lung are within the thoracic cavity. As thoracic cavity at least partially fills with air, the lung will at least partially collapse. This can interfere with normal breathing and can result in respiratory distress.

United States patent application publication US2018271465A1 discloses a biomarker of lung condition can conventionally be obtained using a spirometer. A spirometer provides an estimate of the volume of air expelled by the lungs. This is a rather indirect biomarker of the staging of a lung condition, because a reduction in lung volume may only manifest itself at a point where symptoms are well advanced. A lung condition such as Chronic Obstructive Pulmonary Disorder (COPD) is typically not visible on conventional X-ray attenuation images, because the relevant tissue (alveoli-bearing microstructured lung tissue) contains a lot of air. The X-ray dark- field can successfully indicate microstructure, such as lung alveoli. Therefore, imaging the lungs using the dark-field can provide information on the status of COPD.

### SUMMARY

The invention provides for a method of X-ray imaging, a computer program product, and a medical system in the independent claims. Embodiments are given in the dependent claims.

In one aspect a method of X-ray imaging is disclosed. The method comprises receiving a dark-field X-ray image descriptive of a field of view comprising a lung region of the subject. The method further comprises receiving a transmission X-ray image descriptive of the field of view of the lung region of the subject. The method further comprises receiving a pulmonary segmentation in response to inputting one of the dark-field X-ray image into the transmission X-ray image into a pulmonary segmentation module. The method further comprises constructing pneumothorax image by overlaying the pulmonary segmentation onto the other of the dark-field X-ray image and the transmission X-ray image. The method further comprises providing the pneumothorax image.

In another aspect, a computer program product is disclosed. The computer program product comprises a computer-readable storage medium having machine-executable instructions embodied therewith. The machine-executable instructions are configured to implement a method of X-ray imaging.

In another aspect, a medical system is disclosed. The medical system comprises a memory storing machine-executable instructions and a pulmonary segmentation module. The medical system further comprises a computational system. Execution of the machine-executable instructions causes the computational system to receive a dark-field X-ray image descriptive of a field of view comprising a lung region of the subject. Execution of the machine-executable instructions further causes the computational system to receive a transmission X-ray descriptive of the field of view of the lung region of the subject. Execution of the machine-executable instructions further causes the computational system to receive a pulmonary segmentation in response to inputting one of the dark-field X-ray image and the transmission X-ray image into a pulmonary segmentation module. Execution of the machine-executable instructions further causes the computational system to construct a pneumothorax image by overlaying the pulmonary segmentation onto the other of the dark-field X-ray image and the transmission X-ray image. Execution of the machine-executable instructions further causes the computational system to provide the pneumothorax image.

### BRIEF DESCRIPTION OF THE DRAWINGS

In the following preferred embodiments of the invention will be described, by way of example only, and with reference to the drawings in which:
Fig. 1 illustrates an example of a medical system.
Fig. 2 shows a flow chart which illustrates a method of using the medical system of Fig. 1.
Fig. 3 illustrates a further example of a medical system.
Fig. 4 shows a flow chart which illustrates a method of using the medical system of Fig. 3.
Fig. 5 illustrates a cloud base example of a medical system.
Fig. 6 illustrates one method of constructing a pneumothorax image.
Fig. 7 illustrates a further method of constructing a pneumothorax image.
Fig. 8 illustrates a further method of constructing a pneumothorax image.

### DESCRIPTION OF EMBODIMENTS

Like numbered elements in these figures are either equivalent elements or perform the same function. Elements which have been discussed previously will not necessarily be discussed in later figures if the function is equivalent.

In one aspect, there is a method of X-ray imaging. The method comprises receiving a dark-field X-ray image descriptive of a field of view that comprises a lung region of a subject. This may for example be the thoracic cavity where the lung is located. The method further comprises receiving a transmission X-ray image descriptive of the field of view of the lung region of the subject.

The transmission X-ray image is a conventional X-ray image where the X-rays pass through the subject and are attenuated while passing the subject. For example, an X-ray tube could be set up and directed at the subject and this is then used to either expose a film or a two-dimensional digital detector. The dark-field X-ray image is an X-ray image, but in this case the scattering of the X-rays is detected. An advantage of using a dark-field X-ray image is that it may highlight structural properties, particularly smaller features or features, which may not be visible in a transmission X-ray image. The dark-field X-ray images, for example, may be useful in detecting changes in the lung tissue of the subject, which would be difficult or impossible to image using a transmission X-ray image. Some modem X-ray imaging systems are able to acquire both a dark-field X-ray image and a transmission X-ray image simultaneously, resulting in perfectly registered images.

The method further comprises receiving a pulmonary segmentation in response to inputting one of the dark-field X-ray image and a transmission X-ray image into a pulmonary segmentation module. As used herein, the pulmonary segmentation is used to indicate a segmentation, which is descriptive of the subject's lung. This may be a natural segmentation of the lung itself or may, for example, be a segmentation of the thoracic cavity. The pulmonary segmentation module may be specialized to just segmenting the dark-field X-ray image or the transmission X-ray image or a combination of the two.

One particularly good way of implementing the pulmonary segmentation module would be to use a trained U-Net or ResNet. Manually conducted segmentations could be used to label trial images and used to train the pulmonary segmentation module, for example, using a deep learning technique.

The method further comprises constructing a pneumothorax image by overlaying the pulmonary segmentation onto the other of the dark-field X-ray image and the transmission X-ray image. That is to say, if the dark-field X-ray image is segmented, then the pneumothorax image is constructed by overlaying this segmentation of the dark-field image onto the transmission X-ray image. The other option is that the transmission X-ray image is segmented and then the pneumothorax image is constructed by overlaying this segmentation of the X-ray image onto the dark-field X-ray image. The pneumothorax image, which has the segmentation overlaid on it, is extremely useful because the dark-field X-ray image and the transmission X-ray image, image primarily different types of tissue or show different details. The transmission X-ray image may for example show very good detail in terms of the bone or hard structure of the subject. This may, for example, be useful in determining the boundary of the thoracic cavity, which holds the lung. The dark-field X-ray image on the other hand is better or shows the detail of the soft tissue of the lung. The pneumothorax image may provide a convenient means for a medical technologist or physician to examine the subject.

In another example, the pulmonary segmentation module is configured to output a confidence level of the pulmonary segmentation. The method further comprises modifying the overlay of the pulmonary segmentation using the confidence level. For example, if the pulmonary segmentation module is implemented using a neural network then the neural network may be used to provide, for example, a probability that the segmentation is correct. This confidence level could be output directly by, for example, a U-Net or ResNet. In some examples, this probability might be a global value or in other examples it might be a locally resolved value that changes along the contour of the segmentation.

In another example, the overlay of the pulmonary segmentation comprises any one of the following: assigning a color using the confidence level and assigning a line thickness using the confidence level. Both of these may be useful in transmitting the information about the confidence level within a graphical format. This may, for example, make it easier for the medical technologist or physician to interpret the pneumothorax image.

In another example, the pulmonary segmentation module is a trained neural network. As was mentioned above, the pulmonary segmentation module could be trained to segment the dark-field X-ray image and/or the transmission X-ray image. In general, the pulmonary segmentation module could be trained by having labeled training data. There may be either transmission X-ray images or dark-field X-ray images that are labeled with an existing segmentation. The images could be input into the pulmonary segmentation module and the output could then be compared to the provided segmentation as part of a training scheme, for example, as part of a deep learning training scheme.

In another example, the trained neural network is a U-Net or a ResNet neural network. Both types of these neural networks are very effective at processing images and, for example, providing accurate segmentation.

In another example, the one of the dark-field X-ray image and the transmission X-ray image is the dark-field X-ray image. The other of the dark-field X-ray image and the transmission X-ray image is the transmission X-ray image. In this case, the dark-field X-ray image is input into the pulmonary segmentation module and the pulmonary segmentation is a lung segmentation. The dark-field X-ray image is particularly good at imaging the lung itself. Superimposing the lung segmentation on the transmission X-ray image may then provide a very useful reference in determining if the subject has a collapsed lung or not.

In another example, the one of the dark-field X-ray image and the transmission X-ray image is the transmission X-ray image. The other of the dark-field X-ray image and the transmission X-ray image is the dark-field X-ray image. The pulmonary segmentation is a thoracic cavity segmentation. In this case, the transmission X-ray image is input into the pulmonary segmentation module. As the transmission X-ray image is better at imaging hard and boney type tissue, it is appropriate for accurate segmentation of the thoracic cavity. This thoracic cavity segmentation is then superimposed on the dark-field X-ray image which, for example, shows the actual position of the lung. This may help make an accurate determination if the subject is suffering from a collapsed or partially collapsed lung.

In another example, the method further comprises receiving a second pulmonary segmentation in response to inputting the other of the dark-field X-ray image and the transmission X-ray image into the pulmonary segmentation module. In this case, there may be a single pulmonary segmentation module or the pulmonary segmentation module may comprise sub-pulmonary segmentation modules specialized for the dark-field X-ray image and the transmission X-ray image. The method further comprises calculating a pneumothorax metric descriptive of a distance between the first pulmonary segmentation and the second pulmonary segmentation.

The method further comprises providing a pneumothorax metric. In this example, the two different segmentations are used. There is a segmentation of the dark-field X-ray image, which primarily indicates the location of the lung and the transmission X-ray image, which primarily indicates the position of the thoracic cavity. By measuring a distance between these, or by using a metric which is descriptive of a distance, this may provide an accurate measure if the subject has a collapsed or partially collapsed lung. As the lung collapses the distance between the lung and the thoracic cavity increases.

In another example, the method further comprises providing a warning signal if the pneumothorax metric is above a predetermined threshold. For example, if a maximum distance between the two segmentations is more than a predetermined threshold this may trigger a warning signal. This, for example, could be used to automatically send the pneumothorax image to a particular computer or location. In other cases, it may order or cause instructions to be provided to a healthcare provider on how to attend to the subject.

In another example, the pneumothorax metric is calculated as a maximum of distances between corresponding points on the second pulmonary segmentation and the pulmonary segmentation. Mathematically there are a variety of ways of calculating the maximum of distances between corresponding points and the second pulmonary segmentation. A convenient way of computing this pneumothorax distance would be to calculate the Hausdorff distance between the two segmentations. In the Hausdorff distance for each point on one of the segmentations, one can go through and calculate the smallest distance to the other segmentation or curve. For example, for each point one goes along and finds the nearest point of the other segmentation. To find the maximum, one would then look at each of these minimums and look for the maximum of this. This is mathematically straightforward and could be performed very rapidly.

In another example, the calculation of the pneumothorax metric is limited to lateral regions of the lung or a predetermined region of the lung. For example, the lateral regions of the lung are on the side of the body. The lateral regions of the lung are also typically where the lung detaches or becomes separated from the thoracic cavity. In some instances, it may be useful to limit the calculation of the pneumothorax metric to this region.

In another example, the method further comprises controlling an X-ray system to acquire a dark-field X-ray image and a transmission X-ray image of the field of view of the subject. There are a variety of ways which an X-ray system can be implemented to acquire both dark-field X-ray images and transmission X-ray images. A common way is to use a so-called Talbot-Lau interferometer. In a Talbot-Lau interferometer a series of three different gratings to provide an X-ray interferometer that is able to measure the ultra small angle scattering of X-rays.

In another aspect, there is a computer program product or computer program that comprises a computer-readable storage medium such as a memory or non-transitory storage medium that has machine-executable instructions embodied therewith. The machine-executable instructions are configured to implement an example of the method. This example may be beneficial because it may provide for a means of either updating an existing X-ray system or a system such as a radiology station or cloud-based system to perform examples of the method.

In another aspect, there is a medical system. The medical system comprises a memory storing machine-executable instructions and a pulmonary segmentation module. The memory may, for example, be a non-transitory storage medium. The medical system further comprises a computational system. The computational system may, for example, be one or more processors or computational systems located at one or more locations. In some examples, the computational system may be provided via a cloud-based computing system. Execution of the machine-executable instructions causes the computational system to receive a dark-field X-ray image descriptive of a field of view comprising a lung region of the subject. Execution of the machine-executable instructions further causes the computational system to receive a transmission X-ray image descriptive of a field of view of the lung region of the subject.

Execution of the machine-executable instructions further causes the computational system to receive a pulmonary segmentation in response to inputting one of the dark-field X-ray image and the transmission X-ray image into a pulmonary segmentation module. Execution of the machine-executable instructions further causes the computational system to construct a pneumothorax image by overlaying the pulmonary segmentation onto the other of the dark-field X-ray image and the transmission X-ray image. Execution of the machine-executable instructions further causes the computational system to provide the pneumothorax image. The advantages of this medical system have been previously discussed.

In another example, the medical system further comprises an X-ray system. Execution of the machine-executable instructions further causes the computational system to acquire the dark-field X-ray image and the transmission X-ray image of the field of view of the subject. Advantages of this example have been previously discussed.

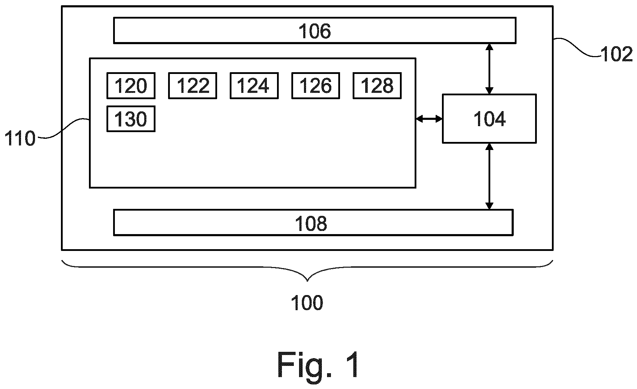

Fig. 1 illustrates an example of a medical system 100. In this example, the medical system 100 comprises a computer 102. The computer 102 may represent one or more computers or computing systems at one or more locations. For example, the functions of the computer 102 could be spread at different locations connected by the internet or other networking systems. The computer 102 is shown as comprising a computational system 104. The computational system 104 may represent one or more computational cores or computational systems that may also be at one or more locations. The computational system 104 is in communication with an optional hardware or network interface that may, for example, be used to communicate with other computer systems and/or to control other components of the medical system 100 if they are present. For example, the hardware interface may be used to control an X-ray system. The computational system 104 is shown as being in further communication with an optional user interface 108. The optional user interface 108 may, for example, be used to display information or images and may also provide a means for an operator to control the operation and function of the medical system 100.

The computational system 104 is shown as being in further communication with a memory 110. The memory 110 is intended to represent various types of memory or storage, which may be accessible to the computational system 104. In some examples, the memory 110 may be a non-transitory storage medium.

The memory 110 is shown as containing machine-executable instructions 120. The machine-executable instructions 120 enable the computational system 104 to perform various tasks such as controlling other components and also performing numerical and image processing tasks. The memory 110 is further shown as containing a pulmonary segmentation module 122. In some examples the pulmonary segmentation module 122 may be configured to receive a transmission X-ray image and output a thoracic cavity segmentation. In other examples, the pulmonary segmentation module 122 may be configured to receive a dark-field X-ray image 126 and output a lung segmentation. In further examples, the pulmonary segmentation module 122 may be a segmentation module, which can produce both types of segmentations for both types of images. In other examples, the pulmonary segmentation module 122 may be a collection of multiple segmentation modules, some configured for the transmission X-ray image 124 and some for the dark-field X-ray image 126.

The memory 110 is shown as containing a transmission X-ray image 124 and a dark-field X-ray image 126 acquired for the same field of view of the subject and being descriptive of the lung region or thoracic cavity of the subject. The memory 110, is shown as containing a pulmonary segmentation 128, which is a segmentation output by the pulmonary segmentation module 122 in response to receiving either the transmission X-ray image 124 or the dark-field X-ray image 126. The memory 110 is further shown as containing a pneumothorax image 130 that was constructed by superimposing the pulmonary segmentation 128 on either the transmission X-ray image 124 or the dark-field X-ray image 126. The image used to construct the pneumothorax image 130 is an image that was not used or input into the pulmonary segmentation module 122.

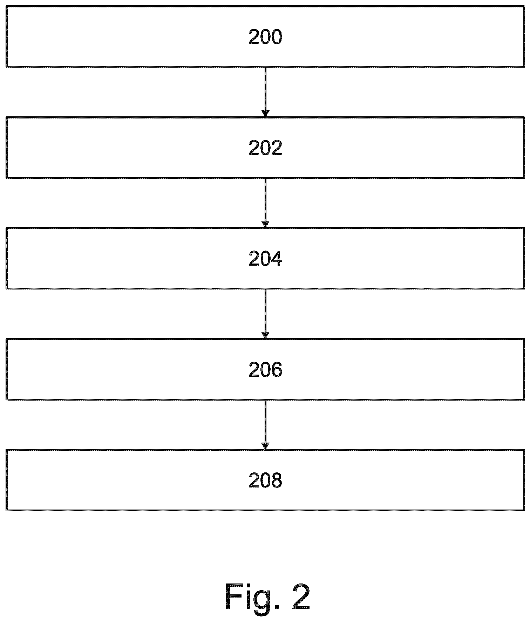

Fig. 2 shows a flowchart which illustrates a method of using the medical system 100 of Fig. 1. In step 200, the dark-field X-ray image 126 is received. This dark-field X-ray image is descriptive of a field of view comprising a lung region of a subject. In step 202, the transmission X-ray image 124 is also received. This transmission X-ray image 124 is for the same field of view as the dark-field X-ray image 126. In step 204, the pulmonary segmentation 128 is received in response to inputting one of the transmission X-ray image 124 and the dark-field X-ray image 126 into the pulmonary segmentation module 122. In step 206, the pneumothorax image 130 is constructed by superimposing the pulmonary segmentation 128 on the other of the transmission X-ray image 124 and the dark-field X-ray image 126. In step 208, the pneumothorax image 130 is provided. This could be provided in different ways, in some examples it may be stored in an image or image database such as attached to a DICOM file. In other examples, the pneumothorax image could be displayed on a display of the user interface 108. In further examples, the pneumothorax image 130 could be transferred or provided to a different computer system, for example, a computer workstation or handheld portable computing device for examination by a medical technician or physician.

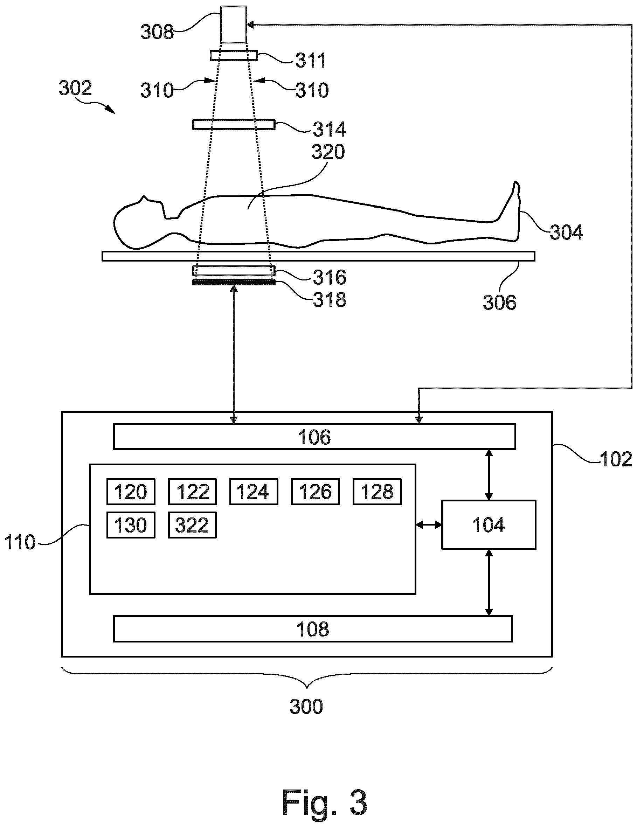

Fig. 3 illustrates a further example of a medical system 300. In this example, the medical system 300 is similar to the medical system 100 depicted in Fig. 1 but additionally comprises an X-ray system 302 or X-ray system. A subject 304 is shown as reposing on a subject support 306. Suspended above the subject 304 is an X-ray source 308. This produces an X-ray beam 310. The X-ray beam 310 then passes through three gratings. There is a source grating 312, which causes the X-ray beam 312 to become a set of partially coherent X-ray line sources. The X-ray beam 310 then passes through a phase grating 314, an analyzer grating 316 and then finally, hits a two-dimensional X-ray detector 318. The subject 304 is placed between the source grating 312 and the phase grating 314, but in alternative embodiments, the subject is placed between the phase grating 314 and the analyzer grating 316. The arrangement illustrated in Fig. 3 is just one way of constructing an X-ray system that is able to acquire dark-field images and also conventional transmission X-rays. The computational system 104 may control the X-ray system 302 such that the phase grating 314 is moved in small steps and the X-ray data 322 is acquired multiple times. In alternative examples, the source grating or the analyzer grating is moved. In yet other alternative examples, multiple gratings are moved.

The X-ray interferometer data 322 thereby consists of a number of phase stepping images acquired for different relative positions of the gratings 312, 314, 316. The transmission X-ray image 124 can be reconstructed by summing the intensity values from all of these phase stepping images in the X-ray interferometer data 322. Alternatively, the gratings 312, 314, and 316 can be removed from the beam path when acquiring the transmission X-ray image 124.

The dark-field image can be reconstructed by analyzing the reduction in fringe visibility in the interference patterns: The scattering of the X-rays causes a reduction in contrast, i.e., the visibility, of the interference fringes. The arrangement depicted in Fig. 3 is a Talbot-Lau interferometer. Other arrangements may also be used to simultaneously acquire transmission X-ray images 124 and dark-field X-ray image 126. The region 320 is the chest region of the subject 304 illuminated by the X-ray beam 310 and is the field of view 320.

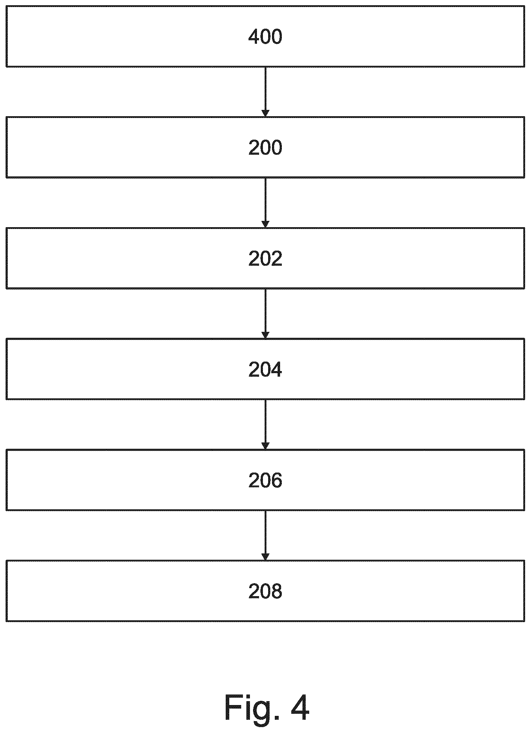

Fig. 4 shows a flowchart, which illustrates a method of operating the medical system 300 of Fig. 3. The method illustrated in Fig. 4 is similar to the method illustrated in Fig. 2. In step 400, the computational system controls the X-ray system 302 to acquire the X-ray interferometer data and to reconstruct the transmission X-ray image 124 and the dark-field X-ray image 126. The method then comprises steps 200, 202, 204, 206, and 208, as was illustrated in Fig. 2.

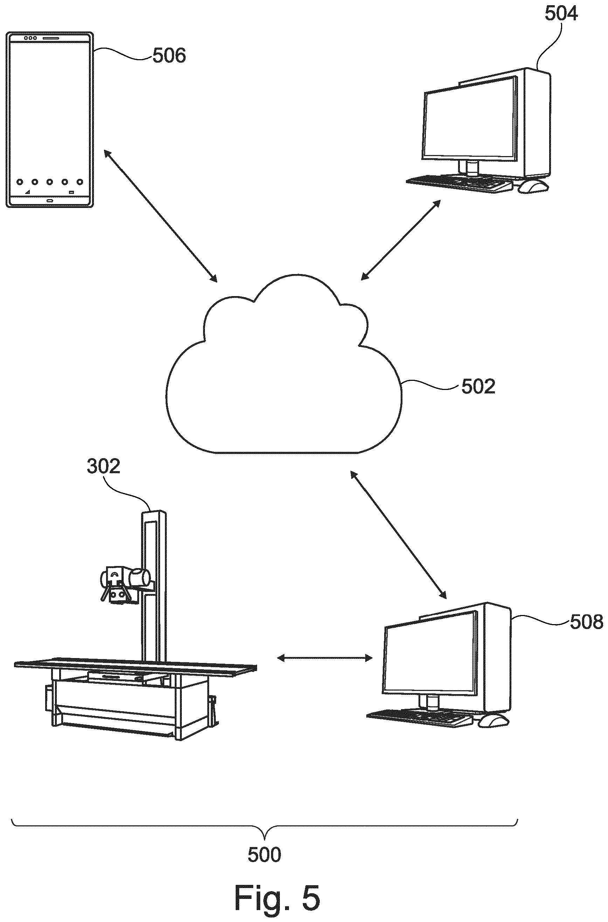

Fig. 5 illustrates an alternative implementation of the medical system 500. This is a cloud-based example. There is a cloud computing system 502, which may for example provide image reconstruction and other numerical tasks. The cloud is shown as being connected to a workstation 504, a handheld telecommunications device 506, and a local controller 508. The computational functions of the computer 102, illustrated in Fig. 3, may be distributed amongst these components 502, 504, 506, and 508.

The local controller 508 may for example be used to control the X-ray system 302. The local controller 508 may then provide the X-ray interferometer data 322 or the transmission X-ray image 124 and the dark-field X-ray image 126 to the cloud 508. In some examples, the cloud 508 would then go through and provide the pneumothorax image 130. The resulting pneumothorax image 130 could for example be displayed using the handheld telecommunications device 506 or on the workstation 504.

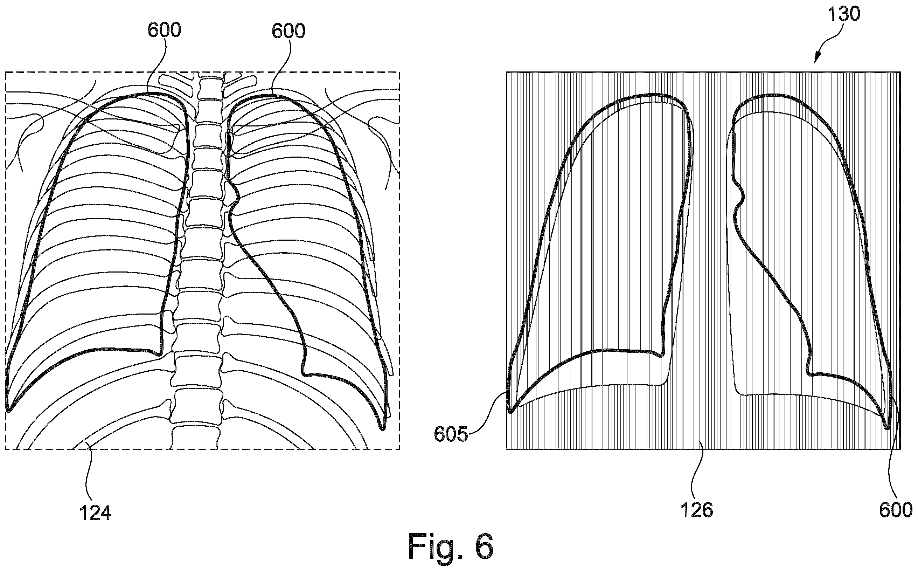

Fig. 6 illustrates one variation of making the pneumothorax image 130. On the left hand side is the standard transmission X-ray image 124. It has been segmented and there is a thoracic cavity segmentation 600. The pneumothorax image 130 is shown on the right side. This image was reconstructed by superimposing the thoracic cavity segmentation 600 over the dark-field X-ray image 126.

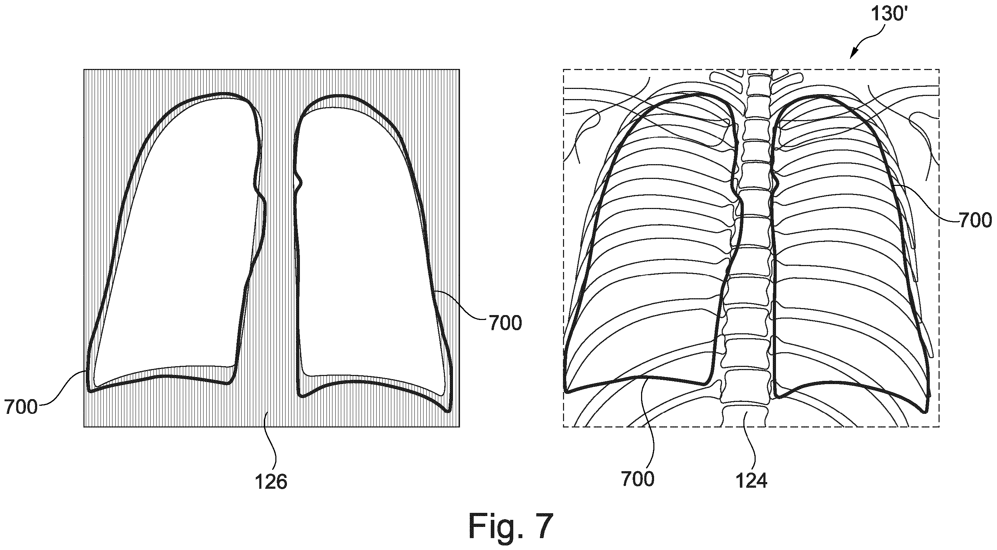

Fig. 7 illustrates a further means of constructing a pneumothorax image 130'. On the left hand side is the dark-field X-ray image 126. It has been segmented and there are two lung segmentations 700 present. On the right hand side is the pneumothorax image 130'. It is constructed by superimposing the lung segmentations 700 on top of the transmission X-ray image 124.

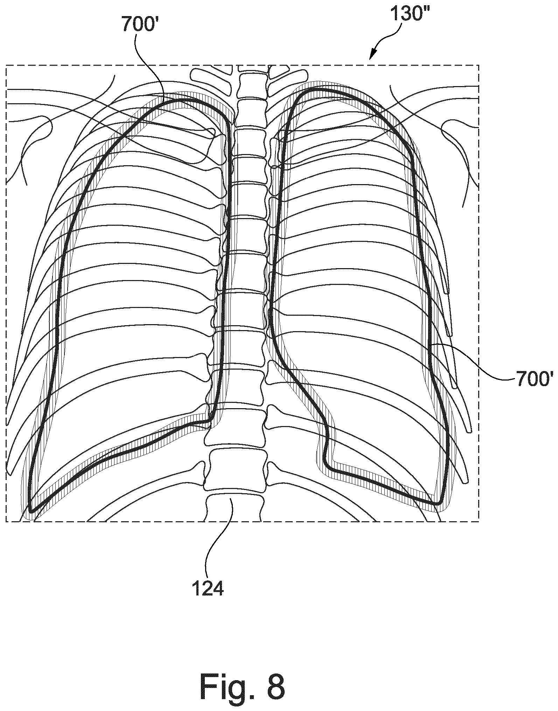

Fig. 8 shows a further modification of the pneumothorax image 130' depicted in Fig. 7. In this example, there is a confidence level, which is provided for the lung segmentation 700. The confidence level can be used to adjust the appearance of the lung segmentation 700 and produce a modified lung segmentation 700'. In this example, the color, opacity, and/or width may be adjusted. The modified pneumothorax image 130" is shown as being constructed from the transmission X-ray image 124 with the modified lung segmentation 700' superimposed on top of it.

It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit", "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A `computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

`Computer memory' or 'memory' is an example of a computer-readable storage medium. Computer memory is any memory which is directly accessible to a computational system. `Computer storage' or 'storage' is a further example of a computer-readable storage medium. Computer storage is any non-volatile computer-readable storage medium. In some embodiments computer storage may also be computer memory or vice versa.

A `computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational systems to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the C programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a Local Area Network (LAN) or a Wide Area Network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a computational system of a general-purpose computer, special-purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.
A `user interface' as used herein is an interface which allows a user or operator to interact with a computer or computer system. A `user interface' may also be referred to as a `human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.
A `hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, WLAN connection, TCP/IP connection, Ethernet connection, control voltage interface, MIDI interface, analog input interface, and digital input interface.

A 'display' or `display device' as used herein encompasses an output device or a user interface adapted for displaying images or data. A display may output visual, audio, and or tactile data. Examples of a display include, but are not limited to: a computer monitor, television screen, touch screen, tactile electronic display, Braille screen, Cathode Ray Tube (CRT), storage tube, bi-stable display, electronic paper, vector display, flat panel display, Vacuum Fluorescent (VD) display, Light-Emitting Diode (LED) displays, Electro-Luminescent Display (ELD), Plasma Display Panels (PDP), Liquid Crystal Display (LCD), Organic Light-Emitting Diode (OLED) displays, a projector, and head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

100 medical system
102 computer
104 computational system
106 hardware / network interface
108 user interface
110 memory
120 machine executable instructions
122 pulmonary segmentation module
124 transmission x-ray image
126 dark-field x-ray image
128 pulmonary segmentation
130 pneumothorax image
130' pneumothorax image
130" pneumothorax image
200 receiving a dark-field x-ray image descriptive of a field of view comprising a lung region of a subject
202 receiving a transmission x-ray image descriptive of the field of view of the lung region of the subject
204 receiving a pulmonary segmentation in response to inputting one of the dark-field x-ray image and the transmission x-ray image into a pulmonary segmentation module
206 constructing pneumothorax image by overlaying the pulmonary segmentation onto the other of the dark-field x-ray image and the transmission x-ray image
208 providing the Pneumothorax image
300 medical system
302 x-ray system
304 subject
306 subject support
308 x-ray source
310 x-ray beam
312 source grating
314 phase grating
316 analyzer grating
318 x-ray detector
320 field of view
322 x-ray interferometer data
400 acquire the dark-field x-ray image and the transmission x-ray image of the field of view of the subject
500 medical system
502 cloud computing system
504 workstation
506 handheld telecommunications device
508 local controller
600 thoracic cavity segmentation
700 lung segmentation
700' modified lung segmentation

## Claims

1. A method of x-ray imaging, wherein the method comprises:
- receiving (200) a dark-field x-ray image (126) descriptive of a field of view (320) comprising a lung region of a subject (304),
- receiving (202) a transmission x-ray image (124) descriptive of the field of view of the lung region of the subject;
- receiving (204) a pulmonary segmentation (128, 600, 700, 700') in response to inputting one of the dark-field x-ray image and the transmission x-ray image into a pulmonary segmentation module (122);
- constructing (206) pneumothorax image (130, 130', 130") by overlaying the pulmonary segmentation onto the other of the dark-field x-ray image and the transmission x-ray image;
- providing (208) the pneumothorax image.

2. The method of claim 1, wherein the pulmonary segmentation module is configured to output a confidence level of the pulmonary segmentation, wherein the method further comprises modifying the overlay of the pulmonary segmentation (700') using the confidence level.

3. The method of claim 2, wherein modifying the overlay of the pulmonary segmentation comprises any one of the following: assigning a color using the confidence level and assigning a line thickness using the confidence level.

4. The method of claim 1, 2, or 3, wherein the pulmonary segmentation module is a trained neural network.

5. The method of claim 4, wherein the trained neural network is a U-Net or a ResNET neural network.

6. The method of x-ray imaging of any one of the preceding claims, wherein the one of the dark-field x-ray image and the transmission x-ray image is the dark-field x-ray image (126), wherein the other of the dark-field x-ray image and the transmission x-ray image is the transmission x-ray image (124), wherein the pulmonary segmentation is a lung segmentation (700).

7. The method of x-ray imaging of any one of claims 1 through 5, wherein the one of the dark-field x-ray image and the transmission x-ray image is the transmission x-ray image (124), wherein the other of the dark-field x-ray image and the transmission x-ray image is the darkfield x-ray image (126), wherein the pulmonary segmentation is a thoracic cavity segmentation (600).

8. The method of x-ray imaging of anyone of the preceding claims, wherein the method further comprises:
- receiving a second pulmonary segmentation in response to inputting the other of the dark-field x-ray image and the transmission x-ray image into the pulmonary segmentation module;
- calculate a Pneumothorax metric descriptive of a distance between the first pulmonary segmentation and pulmonary segmentation; and
- provide the Pneumothorax metric.

9. The method of x-ray imaging of claim 8, wherein the method further comprises providing a warning signal if the Pneumothorax metric is above a predetermined threshold.

10. The method of x-ray imaging of claim 8 or 9, wherein the Pneumothorax metric is calculated as a maximum of distances between corresponding points of the second pulmonary segmentation and the pulmonary segmentation.

11. The method of x-ray imaging of claim 8, 9, or 10, wherein the calculation of Pneumothorax metric is limited to lateral regions of the lung or a predetermined region of the lung.

12. The method of x-ray imaging of any one of the preceding claims, wherein the method further comprises control an x-ray system (302) to acquire (400) the dark-field x-ray image and the transmission x-ray image of the field of view of the subject.

13. A computer program product comprising a computer-readable storage medium having machine executable instructions embodied therewith, said machine executable instructions are configured to implement the method of any one of claims 1 through 12.

14. A medical system comprising:
- a memory storing machine executable instructions and a pulmonary segmentation module;
- a computational system, wherein execution of the machine executable instructions causes the computational system to:
- receive (200) a dark-field x-ray image (126) descriptive of a field of view (320) comprising a lung region of a subject (304),
- receive (202) a transmission x-ray image (124) descriptive of the field of view of the lung region of the subject;
- receive (204) a pulmonary segmentation (128, 600, 700, 700') in response to inputting one of the dark-field x-ray image and the transmission x-ray image into a pulmonary segmentation module (122);
- construct (206) A pneumothorax image (130, 130', 130") by overlaying the pulmonary segmentation onto the other of the dark-field x-ray image and the transmission x-ray image;
- provide (208) the pneumothorax image.

15. The medical system of claim 14, wherein the medical system further comprises an x-ray system (302), wherein execution of the machine executable instructions further causes the computational system to acquire (400) the dark-field x-ray image and the transmission x-ray image of the field of view of the subject.
